# EUROPÄISCHE PATENTSCHRIFT

(11) **EP 0 995 398 B1**
(45) Veröffentlichungstag und Bekanntmachung des Hinweises auf die Patenterteilung: **01.08.2007**
(21) Anmeldenummer: 99203405.8
(22) Anmeldetag: 15.10.1999
(51) Int. Cl.: A61B 6/00, G03B 42/02

(54) **Röntgenuntersuchungsgerät**
X-ray examination apparatus
Appareil d'examen par rayons X

(30) Priorität: 24.10.1998 DE 19849091
(43) Veröffentlichungstag der Anmeldung: 26.04.2000
(73) Patentinhaber: Philips Intellectual Property & Standards GmbH, 20099 Hamburg (DE); Koninklijke Philips Electronics N.V., 5621 BA Eindhoven (NL)
(72) Erfinder: Csikos Janos Philips Corp.Intell. Pro. GmbH, D-52064 Aachen (DE); Medgyesi, György Philips Corp.Intell.Prop.GmbH, D-52064 Aachen (DE); Barnavari, Attila Philips Copr. Intell. Prop. GmbH, D-52064 Aachen (DE)
(74) Vertreter: Volmer, Georg

(56) Entgegenhaltungen:
- EP-A- 0 048 382
- DE-A- 4 210 421
- DE-A- 19 518 572
- GB-A- 2 095 080

## Beschreibung

Die Erfindung betrifft ein Röntgenuntersuchungsgerät mit einem Patientenlagerungstisch, einem in Tischlängsrichtung auf einem ersten Wagen verfahrbaren Durchleuchtungs-Bildwandler, einem darauf ausgerichteten und mit dem ersten Wagen gekoppelten Übertisch-Röntgenstrahler und mit einem Aufnahme-Bildwandler, der auf einem zweiten Wagen verfahrbar ist.

Bekannte Geräte dieser Art haben in der Regel einen Röntgenbildverstärker als Durchleuchtungs-Bildwandler und ein Laufraster zur Aufnahme einer Filmkassette als Aufnahme-Bildwandler. Dabei ist ein schneller Wechsel zwischen einer Aufnahme und einer Röntgendurchleuchtung erforderlich. Während einer Aufnahme befindet sich das Laufraster mit der Filmkassette zwischen dem Röntgenbildverstärker und dem Röntgenstrahler. Bei einer Durchleuchtung hingegen muß die Filmkassette aus dem Strahlengang der Röntgenröhre in eine Parkposition verfahren werden. Zu diesem Zweck ist bei bekannten Röntgenuntersuchungsgeräten der eingangs genannten Art ein in Tischlängsrichtung verfahrbarer erster Wagen vorgesehen, der einen Röntgenbildverstärker und ein Zielgerät trägt, innerhalb dessen ein zweiter Wagen mit der Filmkassette quer zur Tischlängsrichtung verfahrbar ist. Ein derartiges Zielgerät ist einerseits aufwendig und setzt voraus, daß das Röntgenuntersuchungsgerät breit genug ist, um ein seitliches Verfahren des zweiten Wagens in die Parkposition zu ermöglichen.

Aufgabe der vorliegenden Erfindung ist es, mit einfachen Mitteln ein Röntgenuntersuchungsgerät der eingangs genannten Art mit einem geringen Bauvolumen zu schaffen. Diese Aufgabe wird erfindungsgemäß dadurch gelöst, daß der zweite Wagen unabhängig von dem ersten in Tischlängsrichtung verfahrbar ist, und daß ein Antriebssystem vorgesehen ist, das in einem ersten Betriebsmodus die beiden Wagen miteinander synchron und in einem zweiten Betriebsmodus den zweiten relativ zum ersten verfährt.

Bei der Erfindung ist der zweite Wagen unabhängig von dem ersten in Tischlängsrichtung verfahrbar, d.h. die Parkposition ist gegenüber der Aufnahmeposition in Tischlängsrichtung versetzt, so daß die seitlichen Abmessungen des Röntgenuntersuchungsgeräts dadurch nicht verbreitert werden. In dem ersten Betriebsmodus werden die beiden Wagen synchron miteinander verfahren, wobei der zweite in einer definierten Position zum ersten bleibt, z.B. in einer Parkposition unmittelbar neben dem ersten Wagen. Wenn dann eine Röntgenaufnahme angefertigt werden soll, ist im zweiten Betriebsmodus nur eine relativ kurze Verschiebung des zweiten Wagens erforderlich - unabhängig von der Stellung des ersten Wagens.

Das Antriebssystem könnte beispielsweise durch zwei in Tischlängsrichtung wirkende Spindelantriebe realisiert werden. Im ersten Betriebsmodus würden beide Spindelantriebe synchron laufen und dadurch auch die beiden Wagen synchron verschieben, während im zweiten Betriebsmodus der eine Spindelantrieb stillsteht und der zweite Spindelantrieb wirksam ist und den zweiten Wagen verschiebt. Anspruch 2 beschreibt eine noch einfachere Ausgestaltung des Antriebssystems. Unter gegensinniger Änderung der Schleifenlänge wird dabei verstanden, daß die Länge der einen Schleife zunimmt, wenn die Länge der anderen abnimmt - und umgekehrt. Wenn der Antrieb für den ersten Wagen stillsteht und die Trommel rotiert (zweiter Betriebsmodus), verkürzt sich die eine und verlängert sich die andere Schleife, wobei die Umlenkmittel an dem zweiten Wagen diesen relativ zum ersten verschieben. Wenn hingegen im ersten Betriebsmodus der erste Wagen durch den Antrieb verschoben wird und die Trommel stillsteht, kann sich der zweite Wagen relativ zum ersten nicht verschieben, weil dies eine Verlängerung einer der beiden Schleifen voraussetzen würde, was bei stillstehender Trommel nicht möglich ist.

Bei der in Anspruch 4 angegebenen weiteren Ausgestaltung kann ein Patient in dem ganzen Bereich zwischen den beiden Enden des Rahmens untersucht werden, d.h. daß der Durchleuchtungs-Bildwandler vom Kopf bis zum Fußende verschoben werden kann.

Grundsätzlich könnten zwei Zugmittel vorgesehen sein, die mit ihren Enden an der Trommel befestigt und gegensinnig auf diese aufgewickelt sind, sodaß sich das eiene Zugmittel bei einer Drehung der Trommel auf- und das andere abwickelt. Die Ausgestaltung nach Anspruch 5 ist demgegenüber jedoch einfacher, weil sie nur ein einziges Zugmittel voraussetzt. Die Ausgestaltung nach Anspruch 6 stellt dabei sicher, daß das Zugmittel überall außerhalb der Umlenkrollen parallel zur Tischlängsrichtung verläuft.

Bei einem Röntgenuntersuchungsgerät, bei dem der Aufnahme-Bildwandler in einem Zielgerät quer zur Tischlängsrichtung verfahren wird, kann durch eine fest mit dem Zielgerät verbundene, für Röntgenstrahlung undurchlässige Abschirmung der Aufnahme-Bildwandler gegen die bei einer Durchleuchtung vor oder nach der Aufnahme entstehende Streustrahlung abgeschirmt werden. Bei einem Gerät, bei dem die Parkposition in Tischlängsrichtung versetzt ist, kann der Aufnahme-Bildwandler in der Parkposition nicht durch einen stationären Schirm gegen die bei der Durchleuchtung entstehende Streustrahlung abgeschirmt werden, weil ein solcher Schirm den Bereich begrenzen würde innerhalb dessen eine Durchleuchtung oder eine Aufnahme möglich ist. Die in Anspruch 3 beschriebene Ausgestaltung erlaubt einen Streustrahlenschutz mit einem quer zur Tischlängsrichtung verfahrbaren Streustrahlenschirm.

Bei der weiteren Ausgestaltung nach Anspruch 7 wird - ohne einen gesonderten Antrieb für den Streustrahlenschirm - sichergestellt, daß der Streustrahlenschirm den Bildaufnehmer immer dann abschirmt, wenn dieser sich in einer Parkposition befindet und ihn freigibt, wenn er sich in der Aufnahmeposition befindet und auf den Durchleuchtungs-Bildaufnehmer ausgerichtet ist.

Anspruch 8 beschreibt eine als Teil eines Röntgenuntersuchungsgerätes geeigneten erfindungsgemäß ausgestalteten Patientenlagerungstisch.

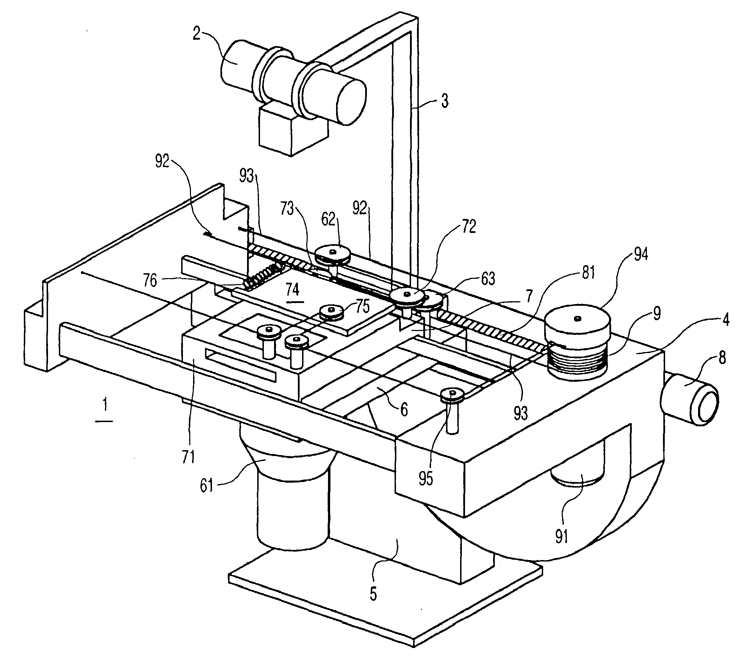

Die Erfindung wird nachstehend anhand der Zeichnung näher erläutert. Das Röntgenuntersuchungsgerät umfaßt einen Patientenlagerungstisch 1 und einen Röntgenstrahler 2, der an einem in Tischlängsrichtung verfahrbaren Stativ 3 oberhalb des Patientenlagerungstisches 1 befestigt ist und daher auch als Übertisch-Röntgenstrahler bezeichnet wird. Der Patientenlagerungstisch 1 ist ohne eine Tischplatte dargestellt, so daß man seine darunter befindlichen Komponenten erkennen kann.

Der Patientenlagerungstisch weist einen Rahmen 4 auf, der an einer Tischbasis 5 um eine horizontale Achse schwenkbar gelagert ist, so daß er aus der dargestellten horizontalen Lage in eine geneigte oder in eine vertikale Lage geschwenkt werden kann. In dem Rahmen 4 ist ein erster Wagen 6 in Tischlängsrichtung verfahrbar. Dieser Wagen trägt einen RöntgenBildverstärker 61. Oberhalb des ersten Wagens befindet sich ein zweiter Wagen 7, der unabhängig von dem ersten ebenfalls in Längsrichtung des Tisches verschiebbar ist. Der zweite Wagen weist ein Laufraster 71 auf, mit einem Schlitz zur Einführung bzw. zur Entnahme einer Filmkassette.

Der erste Wagen 6 kann durch einen ersten Motor 8 über eine Antriebsspindel 81 verfahren werden. Da der Wagen 6 mit dem Stativ 3 gekoppelt ist, wird der Röntgenstrahler 2 mitgeführt, so daß er stets auf den ersten Wagen 6 bzw. auf den darunter befindlichen Röntgenbildverstärker 61 ausgerichtet bleibt. Der zweite Wagen 7 kann aus seiner in der Zeichnung dargestellten, auf den Bildverstärker 61 bzw. den Röntgenstrahler 2 zentrierten Aufnahmeposition in eine Parkposition - und zurück - mittels einer Trommel 9 verfahren werden, die am fußseitigen Ende des Rahmens 4 befestigt ist und von einem Antriebsmotor 91 angetrieben wird.

Auf die Trommel 9 ist ein Drahtseil gewickelt, dessen Mitte an der Trommel 9 fixiert ist. Das obere rechte Ende 92 des Seils ist über eine erste Umlenkrolle 62 auf der linken (von der Trommel 9 abgewandten) Seite des Wagens 6 und über eine Umlenkrolle 72, die auf der rechten (der Trommel 9 zugewandten) Seite des Wagens 7 befestigt ist, zu dem kopfseitigen Ende des Rahmens 4 geführt und dort befestigt. Das zweite (untere) Ende 93 des Seils ist über eine zweite Umlenkrolle 73 auf der linken Seite des zweiten Wagens 7 und eine zweite Rolle auf der rechten Seite des ersten Wagens 63 zu dem kopfseitigen Ende des Rahmens geführt und ebenfalls dort befestigt.

Durch die Führung der Seilenden über die Umlenkrollen bildetjedes Seilende eine S-förmige Schleife. Da das zweite Seilende 93 jedoch - im Gegensatz zum ersten Seilende 92 - zunächst über eine Rolle (73) am zweiten Wagen und dann erst über eine Rolle (63) am ersten Wagen geführt ist, ist diese S-Form spiegelverkehrt zu der durch das erste Seilende 92 gebildeten S-Form. Deshalb können sich die Längen der Schleifen nur gegensinnig ändern, d.h. bei einer Verschiebung des zweiten Wagens relativ zum ersten Wagen verkürzt sich die eine Schleife und verlängert sich die andere.

Wenn die Trommel 9 im Uhrzeigersinn angetrieben wird, verkürzt sich das Seilende 92 und die darin enthaltene Schleife, während sich das Seilende 93 und die darin enthaltene Schleife verlängern. Infolgedessen verschiebt sich der zweite Wagen relativ zum ersten Wagen 6 in Richtung des Kopfendes. - Wenn der Motor 91 die Trommel 9 im Gegenuhrzeigersinn antreibt, ist es genau umgekehrt: das Seilende 93 und die darin enthaltene Schleife verkürzen sich, während sich das Seilende 92 und dessen Schleife verlängern. Der zweite Wagen 7 wird dann relativ zum ersten in Richtung auf das Fußende gezogen. Da somit der zweite Wagen relativ zum ersten sowohl in Richtung des Kopfendes als auch in Richtung des Fußendes verschoben werden kann, ist es möglich, den zweiten Wagen in eine kopfseitige Parkposition zu verschieben, wenn der erste Wagen näher am Fußende ist und in eine fußseitige Parkposition, wenn der erste Wagen näher am Kopfende ist. Es ist somit möglich, den Bildverstärker und den Röntgenstrahler in dem gesamten Bereich zwischen den beiden Rahmenenden zu verschieben, so daß ein Patient praktisch über seine gesamte Länge untersucht werden kann, ohne das die nicht dargestellte Tischplatte, auf der er sich befindet, verschoben werden muß.

Wenn der Motor 8 den ersten Wagen 6 antreibt und wenn die Trommel 9 stillsteht, weil der Motor 91 eine Drehung blockiert, dann verschiebt sich der Wagen 6. Wegen der blockierten Trommel 9 verändert sich dabei die Länge der Seilenden 92, 93 nicht, ebenso wenig kann sich die Länge der durch diese Seilenden gebildeten Schleifen verändern, so daß der zweite Wagen 7 dem ersten Wagen 6 folgt, wobei sich die Schleifen in den Seilenden zusammen mit den beiden Wagen 6, 7 verschieben. Dies gilt sowohl dann, wenn der Wagen 7 - wie in der Zeichnung dargestellt - auf den Bildverstärker zentriert ist, als auch dann, wenn er sich in einer Parkposition befindet.

Somit bewegen sich die beiden Wagen bei einem Antrieb durch den Motor 8 und feststehendem Motor 91 synchron miteinander, d.h. sie verändern ihre Lage relativ zueinander nicht (erster Betriebsmodus). Bei einem Antrieb durch den Motor 91 und bei feststehendem Motor 8 bewegt sich hingegen nur der zweite Wagen 7 relativ zum ersten Wagen 6(zweiter Betriebsmodus).

Ein Vorteil des erfindungsgemäßen Röntgengerätes (gegenüber einem Röntgengerät mit fest mit dem Bildverstärkerwagen gekoppelten Zielgerät) besteht darin, daß der zweite Wagen 7 eine eigene Führung am Rahmen 4 hat und daher nicht vom ersten Wagen getragen werden muß. Der Wagen 6 kann daher verhältnismäßig leicht und kompakt aufgebaut sein. Ein anderer Vorteil ist, daß die Antriebsmotoren 8, 91 am Rahmen befestigt sind und nicht auf einem der Wagen 6 bzw. 7.

Wenn sich der Wagen 7 und mit ihm das Laufraster 71 in einer Parkposition befinden, in der sie den Strahlengang auf den Bildverstärker 61 freigeben, besteht die Gefahr, daß eine Filmkassette in dem Laufraster von der bei einer Durchleuchtung in dem Patienten entstehenden Streustrahlung getroffen wird. Diese Streustrahlung kann durch einen für die Röntgenstrahlung undurchlässigen Streustrahlenschirm 74 absorbiert werden, der oberhalb des Laufrasters 71 mit dem Wagen 7 mitgeführt wird und einen im Laufraster enthaltenen Film 71 in den Parkpositionen abschirmt, ihn aber in der dargestellten Aufnahmeposition freigibt.

Zum Verfahren des Streustrahlenschirms ist auf der Antriebsachse der Trommel 9 eine Hilfstrommel 94 vorgesehen, die einen nicht näher dargestellten Schlitz aufweist, in den ein Drahtseil 95 eingeklemmt ist. Dieses ist über eine Umlenkrolle am Rahmen 95, eine erste Umlenkrolle am Laufraster 71, eine Umlenkrolle 75, die an dem Schirm 74 befestigt ist und eine zweite Rolle am Laufraster zum kopfseitigen Ende des Rahmens 4 geführt und dort befestigt.

Das Seil 95 ist vollständig von der Hilfstrommel 94 abgewickelt, wenn die Trommel 9 eine Position einnimmt, in der der zweite Wagen 7 auf den ersten Wagen 6 zentriert ist. Der Streustrahlenschirm 74 wird dann von einer Zugfeder 76 seitlich (d.h. senkrecht zur Tischlängsrichtung) aus dem Strahlengang des Röntgenstrahlers 2 herausgezogen, so daß eine Röntgenaufnahme erfolgen kann. Zur Verschiebung des Wagens 7 in eine Parkposition wird die Trommel 9 und mit ihr die Hilfstrommel 94 gedreht, wobei sich das Seil 95 entweder im Uhrzeigersinn oder im Gegenuhrzeigersinn auf die Hilfstrommel aufwickelt. Dadurch verkürzt sich die Schleife um die Umlenkrolle 75 auf dem Streustrahlenschirm 74, so daß dieser entgegen der Kraft der Feder 76 senkrecht zur Tischlängsrichtung in den Strahlengang gezogen wird, so daß er eine in dem Laufraster 71 befindliche Filmkassette gegen Streustrahlung abschirmen kann.

Die Position des ersten Wagens 6 kann durch einen mit dem Motor 8 gekoppelten elektrischen Geber bestimmt werden. Desgleichen kann die Position des zweiten Wagens 7 relativ zum ersten Wagen 6 mit einem elektrischen Geber bestimmt werden, der mit dem Antriebsmotor 91 gekoppelt ist. Damit ist es möglich, den zweiten Wagen 7 jeweils in die Parkposition zu verfahren, in der er die Verschiebung des ersten in Richtung auf das jeweils nähere Ende des Rahmens nicht behindern kann. - Neben den beiden Betiebsmodi, in denen jeweils nur einer der beiden Motoren 8, 91 wirksam ist, kann ein weiterer Betriebsmodus vorgesehen sein, in dem beide Motoren gleichzeitig wirksam sind. Es werden dann gleichzeitig der erste Wagen 6 in Bezug auf den Rahmen 4 als auch der zweite Wagen 7 relativ zum ersten verschoben werden.

## Patentansprüche

1. Röntgenuntersuchungsgerät mit einem Patientenlagerungstisch (1), einem in Tischlängsrichtung auf einem ersten Wagen (6) verfahrbaren Durchleuchtungs-Bildwandler (61), einem darauf ausgerichteten und mit dem ersten Wagen gekoppelten Übertisch-Röntgenstrahler (2), und mit einem Aufnahme-Bildwandler (71), der auf einem zweiten Wagen (7) verfahrbar ist,
**dadurch gekennzeichnet, daß** der zweite Wagen (7) unabhängig von dem ersten in Tischlängsrichtung verfahrbar ist, und daß ein Antriebssystem (8,81,9,91) vorgesehen ist, das in einem ersten Betriebsmodus die beiden Wagen (6, 7) miteinander synchron und in einem zweiten Betriebsmodus den zweiten Wagen (7) relativ zum ersten Wagen (6) verfährt.

2. Röntgenuntersuchungsgerät nach Anspruch 1,
**dadurch gekennzeichnet, daß** das Antriebssystem einen im ersten Betriebsmodus wirksamen Antrieb (8, 81) zum Verfahren des ersten Wagens (6) und eine im zweiten Betriebsmodus rotierende erste Trommel (9) mit mindestens einem Zugmittel aufweist, das auf die erste Trommel auf- bzw. abwickelbar sind und über Umlenkmittel (62....73) am ersten und am zweiten Wagen in einer ersten und zweiten Schleife geführt und an einem Ende des Patientenlagerungstisches (1) befestigt ist, wobei sich die Länge der Schleifen bei einer Drehung der ersten Trommel (9) gegensinnig ändert.

3. Röntgenuntersuchungsgerät nach Anspruch 1,
**dadurch gekennzeichnet, daß** auf dem zweiten Wagen (7) - quer zur Tischlängsrichtung verschiebbar - ein Streustrahlenschirm (74) angeordnet ist, der so mit dem Antriebssystem gekoppelt ist, daß er den Aufnahme-Bildwandler (71) abschirmt, wenn der zweite Wagen (7) auf den ersten Wagen (6) zentriert ist, und daß er den Aufnahme-Bildwandler freiläßt, wenn der zweite Wagen sich in einer Parkposition seitlich neben dem ersten Wagen befindet.

4. Röntgenuntersuchungsgerät nach Anspruch 2,
**dadurch gekennzeichnet, daß** die erste Trommel (9) einerseits und das (die) Zugmittel (92,93) andrerseits an in Längsrichtung gegenüberliegenden Enden des Patientenlagerungstisches (1) befestigt sind.

5. Röntgenuntersuchungsgerät nach Anspruch 2,
**dadurch gekennzeichnet, daß** ein einziges Zugmittel vorgesehen ist, dessen Enden (92,93) über die Schleifen mit dem Patientenlagerungstisch verbunden sind und das in seinem mittleren Bereich auf die erste Trommel (9) aufgewickelt und mit dieser verbunden ist.

6. Röntgenuntersuchungsgerät nach Anspruch 5,
**dadurch gekennzeichnet, daß** die Enden (92;93) des Zugmittels über je eine Umlenkmittel (62,72; 63,73) an dem ersten und an dem zweiten Wagen geführt, daß die Summe der Durchmesser der Umlenkmittel (62,72; 63,73) dem Durchmesser der ersten Trommel (9) entspricht und daß die Enden des Zugmittels so geführt sind, daß das sich außerhalb der Umlenkmittel ein paralleler Verlauf in Tischlängsrichtung ergibt.

7. Röntgenuntersuchungsgerät nach Anspruch 3,
**dadurch gekennzeichnet, daß** eine zweite Trommel (94) vorgesehen ist, die mit der ersten Trommel (9) gekoppelt ist und auf die ein Zugmittel (95) wickelbar, das in einer Schleife, über Umlenkmittel (75) an dem zweiten Wagen (7) und an dem Streustrahlenschirm (74) geführt und mit dem Rahmen verbunden ist, derart daß die Schleife ihre größte Länge hat, wenn der zweite Wagen (7) auf den ersten Wagen (6) ausgerichtet ist, und daß die Schleife sich verkürzt, wenn sich der zweite Wagen in einer Parkposition befindet.

8. Patientenlagerungstisch (1) für ein Röntgenuntersuchungsgerät, mit einem in Tischlängsrichtung verfahrbaren ersten Wagen (6), der mit einem Übertisch-Röntgenstrahler (2) koppelbar ist und an den ein Durchleuchtungs-Bildwandler (61) montierbar ist, und mit einem Aufnahme-Bildwandler (71), der auf einem zweiten Wagen (7) verfahrbar ist,
**dadurch gekennzeichnet, daß** der zweite Wagen (7) unabhängig von dem ersten in Tischlängsrichtung verfahrbar ist, und daß ein Antriebssystem (8,81,9,91) vorgesehen ist, das in einem ersten Betriebsmodus die beiden Wagen miteinander synchron und in einem zweiten Betriebsmodus den zweiten relativ zum ersten verfährt.

## Claims

1. An X-ray examination apparatus which includes a patient table (1), a fluoroscopy image converter (61) which is mounted on a first carriage (6) so as to be displaceable in the longitudinal direction of the table, an overtable X-ray source (2) which is aligned with respect to said converter and is coupled to the first carriage, and an image recording means (71) which is displaceable on a second carriage (7),
**characterized in that** the second carriage (7) is displaceable in the longitudinal direction of the table, independently of the first carriage, and that there is provided a drive system (8, 81, 9, 91) which displaces the two carriages (6, 7) in synchronism in a first mode of operation and displaces the second carriage (7) relative to the first carriage (6) in a second mode of operation.

2. An X-ray examination apparatus as claimed in Claim 1,
**characterized in that** the drive system includes a drive (8, 81) which is active in the first mode of operation so as to displace the first carriage (6), and also includes a first drum (9) which rotates in the second mode of operation and is provided with at least one pulling means which can be wound onto and from the first drum and is guided, via guide means (62 ... 73) on the first and the second carriage, in a first and a second loop, said pulling means being attached at one end of the patient table (1), the length of the loops changing in opposite directions upon rotation of the first drum (9).

3. An X-ray examination apparatus as claimed in Claim 1,
**characterized in that** on the second carriage (7) there is mounted a scattered radiation shield (74) which is displaceable in the direction transversely of the longitudinal direction of the table and is coupled to the drive system in such a manner that it shields the image recording means (71) when the second carriage (7) is centered with respect to the first carriage (6), and that it leaves the image recording means free when the second carriage is in a parking position to the side of the first carriage.

4. An X-ray examination apparatus as claimed in Claim 2,
**characterized in that** the first drum (9) on the one side and the pulling means (92, 93) on the other side are attached to opposite ends of the patient table (1), viewed in the longitudinal direction.

5. An X-ray examination apparatus as claimed in Claim 2,
**characterized in that** there is provided a single pulling means whose ends (92, 93) are connected to the patient table via the loops, the central part of said pulling means being wound on and attached to the first drum (9).

6. An X-ray examination apparatus as claimed in Claim 5,
**characterized in that** the ends (92; 93) of the pulling means are guided via respective guide means (62, 73; 63, 73) on the first and the second carriage, that the sum of the diameters of the guide means (62, 72; 63, 73) corresponds to the diameter of the first drum (9), and that the ends of the pulling means are guided in such a manner that outside the guide means a parallel trajectory is obtained in the longitudinal direction of the table.

7. An X-ray examination apparatus as claimed in Claim 3,
**characterized in that** there is provided a second drum (94) which is coupled to the first drum (9) and on which there can be wound a pulling means (95) which is guided in a loop via guide means (75) on the second carriage (7) and on the scattered radiation shield (74) and is connected to the frame in such a manner that the length of the loop is greatest when the second carriage (7) is aligned with respect to the first carriage (6), the length of the loop being shorter when the second carriage is in a parking position.

8. A patient table (1) for an X-ray examination apparatus, including a first carriage (6) which is displaceable in the longitudinal direction of the table, can be coupled to an overtable X-ray source (2), and on which a fluoroscopy image converter (61) can be mounted, and also including an image recording means (71) which is displaceable on a second carriage (7),
**characterized in that** the second carriage (7) is displaceable in the longitudinal direction of the table, independently of the first carriage, and that there is provided a drive system (8, 81, 9, 91) which displaces the two carriages in synchronism in a first mode of operation and displaces the second carriage relative to the first carriage in a second mode of operation.

## Revendications

1. Appareil d'auscultation par rayons X muni d'une table de logement du patient (1), d'un convertisseur de radiographie (61) mobile sur un premier chariot (6) dans la direction de la longueur de la table, d'un ensemble radiogène à rayons X (2) situé au-dessus de la table, aligné sur le convertisseur et couplé au premier chariot, et d'un convertisseur d'image enregistreur (71) qui est mobile sur un second chariot (7),
**caractérisé en ce que** le second chariot (7) est mobile dans la direction de la longueur de la table indépendamment du premier chariot, et **en ce qu'**un système d'entraînement (8,81,9,91) est prévu, qui, dans un premier mode de fonctionnement, déplace les deux chariots (6, 7) l'un avec l'autre de manière synchrone, et, dans un second mode de fonctionnement, déplace le second chariot (7) relativement au premier chariot (6).

2. Appareil d'auscultation par rayons X selon la revendication 1,
**caractérisé en ce que** le système d'entraînement présente un entraînement (8, 81) actif dans le premier mode de fonctionnement pour le déplacement du premier chariot (6) et un premier tambour (9) tournant dans le second mode de fonctionnement, muni d'au moins un moyen de traction, qui peut (peuvent) être enroulé(s) ou bien déroulé(s) sur le premier tambour et qui est (sont) guidé(s) jusqu'au premier et jusqu'au second chariot dans une première et une seconde coulisse par l'intermédiaire de moyens de renvoi (62...73), et qui est (sont) fixé(s) à une extrémité de la table de logement du patient (1), où la longueur des coulisses se modifie en sens inverse lors d'une rotation du premier tambour (9).

3. Appareil d'auscultation par rayons X selon la revendication 1,
**caractérisé en ce que**, sur le second chariot (7), un écran contre les rayons diffusés (74) - déplaçable transversalement par rapport à la direction de la longueur de la table - est disposé, écran qui est couplé au système d'entraînement de façon qu'il protège le convertisseur d'image enregistreur (71) lorsque le second chariot (7) est centré sur le premier chariot (6), et qu'il libère le convertisseur d'image enregistreur lorsque le second chariot se trouve dans une position de stationnement latérale à côté du premier chariot.

4. Appareil d'auscultation par rayons X selon la revendication 2,
**caractérisé en ce que** le premier tambour (9), d'une part, et le (les) moyen(s) de traction (92,93), d'autre part, est (sont) fixé(s) à des extrémités opposées de la table de logement du patient (1) dans la direction de la longueur.

5. Appareil d'auscultation par rayons X selon la revendication 2,
**caractérisé en ce qu'**un seul moyen de traction est prévu, dont les extrémités (92,93) sont reliées à la table de logement du patient par l'intermédiaire des coulisses, et qui, en son milieu, est enroulé sur le premier tambour (9) et relié à celui-ci.

6. Appareil d'auscultation par rayons X selon la revendication 5,
**caractérisé en ce que** chacune des extrémités (92;93) du moyen de traction est guidée jusqu'au premier et jusqu'au second chariot par l'intermédiaire d'un moyen de renvoi (62,72; 63,73), **en ce que** la somme des diamètres des moyens de renvoi (62,72; 63,73) correspond au diamètre du premier tambour (9) et **en ce que** les extrémités du moyen de traction sont guidées de façon à obtenir, en dehors des moyens de renvoi, un parcours parallèle dans la direction de la longueur de la table.

7. Appareil d'auscultation par rayons X selon la revendication 3,
**caractérisé en ce qu'**un second tambour (94) est prévu, qui est couplé au premier tambour (9) et sur lequel un moyen de traction (95) peut être enroulé, qui est guidé dans une coulisse jusqu'au second chariot (7) et jusqu'à l'écran contre les rayons diffusés (74) par l'intermédiaire de moyens de renvoi (75), et qui est relié au bâti, de telle façon que la coulisse ait sa plus grande longueur lorsque le second chariot (7) est aligné sur le premier chariot (6), et que la coulisse se raccourcisse lorsque le second chariot se trouve dans une position de stationnement.

8. Table de logement du patient (1) pour un appareil d'auscultation par rayons X, munie d'un premier chariot (6) mobile dans la direction de la longueur de la table, chariot qui peut être couplé à un ensemble radiogène à rayons X (2) situé au-dessus de la table et sur lequel un convertisseur de radiographie (61) peut être monté, et munie d'un convertisseur d'image enregistreur (71), qui est mobile sur un second chariot (7),
**caractérisée en ce que** le second chariot (7) peut se déplacer dans la direction de la longueur de la table indépendamment du premier chariot, et **en ce qu'**un système d'entraînement (8,81,9,91) est prévu, qui dans un premier mode de fonctionnement, déplace les deux chariots l'un avec l'autre de manière synchrone et, dans un second mode de fonctionnement, déplace le second chariot relativement au premier.
